(19) Europäisches Patentamt
European Patent Office
Office européen des brevets

(11) Publication number: **0 200 794 A1**

(12) **EUROPEAN PATENT APPLICATION**

(21) Application number: **85104273.9**

(22) Date of filing: **09.04.85**

(51) Int. Cl.⁴: **C 07 C 175/00**

(43) Date of publication of application: **12.11.86 Bulletin 86/46**

(84) Designated Contracting States: **AT BE CH DE FR GB LI LU NL SE**

(71) Applicant: **MAGIS FARMACEUTICI Spa, via Cacciamali 34/36/38, Brescia (IT)**

(72) Inventor: **Puricelli, Laura, via Taramelli, 7, Brescia (IT)**

(74) Representative: **Gervasi, Gemma, Dr. et al, Studio Brevetti e Marchi NOTARBARTOLO & GERVASI 33, Viale Bianca Maria, I-20122 Milano (IT)**

(54) **Derivatives of ubiquinone, process for their preparation and pharmaceutical compositions containing them.**

(57) New derivatives of ubiquinone (10) are disclosed, of general formula (I):

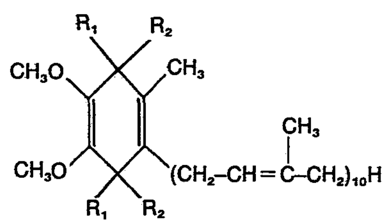

(I)

wherein $R_1$ is hydrogen and $R_2$ means $SO_3Na$, or $R_1$ and $R_2$ together mean the residue of an aminoacid selected from the group constituted by glycine, L-leucine, phenyl-glycine, phenylalanine, aspartic acid or taurine, generally in the form of an alkaline salt thereof, preferably of their sodium salt. A process is also disclosed for the preparation of these new derivatives, as well as pharmaceutical preparations containing them.

The new derivatives of general formula (I) are indicated in the treatment of hypertension, or for relieving advanced hypertension.

EP 0 200 794 A1

"DERIVATIVES OF UBIQUINONE, PROCESS FOR THEIR PREPARATION AND PHARMACEUTICAL COMPOSITIONS CONTAINING THEM"

Disclosure

Ubiquinone(10), also known as Coenzyme $Q_{10}$, and with structure analogous to that of Vitamin $K_2$, is a cardiovascular agent known since long time, provided with a very low-level toxicity (see e.g. "The Vitamins", W.H. Sebrell and R.S.Harris, pages 355-391, Academic Press, New York,1972). It has been now found that by suitably replacing the two ketogroups in the 1- and 4-positions of the benzene ring of ubiquinone with suitable groups, novel derivatives can be obtained, which can also be administred by rectal way or can be injected and which moreover, in some cases, show a much greater light stability. By aqueous, acid or alkaline hydrolysis, these new derivatives yield easily back again the ubiquinone from which they derive.
An object of the present invention are therefore new derivatives of general formula (I)

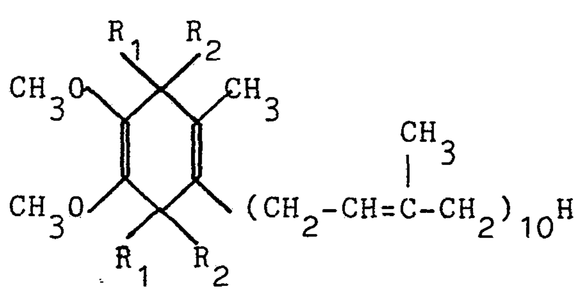

(I)

wherein $R_1$ is hydrogen and $R_2$ means $SO_3Na$, or $R_1$ and $R_2$ together mean the residue of an aminoacid selected from

the group constituted by glycine, L-leucine, phenylglycine, phenylalanine, aspartic acid or taurine, generally in the form of an alkaline salt thereof, preferably of their sodium salt.

These new derivatives of formula (I) are outstandingly interesting from the therapeutical viewpoint; as they are obtained by means of the reaction of ubiquinone with amino acids, they can be injected yielding higher and faster levels than when administred by the oral way.

The disulphite derivative (I: $R_1$ = H; $R_2$ = $SO_3Na$) shows moreover a high stability so that in the oral preparations no decompositions occur under the effect of the light.

An object of the present invention are moreover the pharmaceutical formulations with anti-hypertensive action containing, as their active principle, one or more compound(s) of general formula (I).

An object of the invention is finally also a process for the preparation of the compounds of general formula (I), wherein ubiquinone(10) is reacted in an apolar solvent, and under refluxing conditions, with an alkaline salt, preferably with the sodium salt, of an aminoacid selected from the group constituted by glycine, L-leucine, phenyl-glycine, phenylalanine, aspartic acid and taurine.

The reaction between ubiquinone and aminoacid is made proceed over 20-30 minutes, the reaction mixture is then cooled and filtered.

The precipitate formed is washed with a suitable solvent, preferably a lower aliphatic alcohol, and then with an ether, dried and finally crystallized.

The derivative with the bisulphite is obtained by means of the simple mixing of a solution of sodium bisulphite with ubiquinone(10).

The preparation of the new derivatives of general formula (I) can be schematically shown as follows:

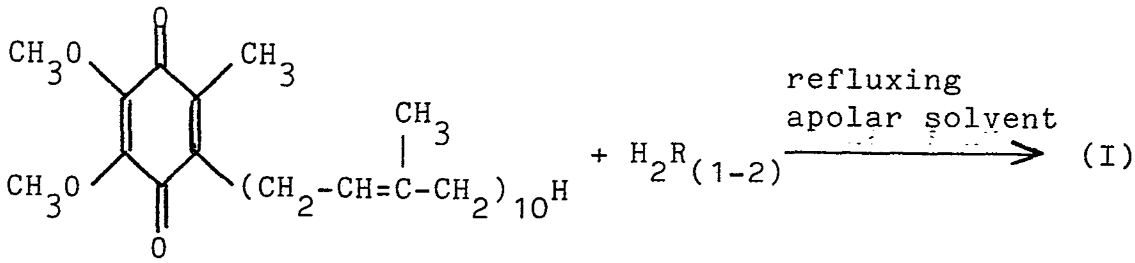


wherein ($R_1$-$R_2$) has the meaning hereinabove shown, or:

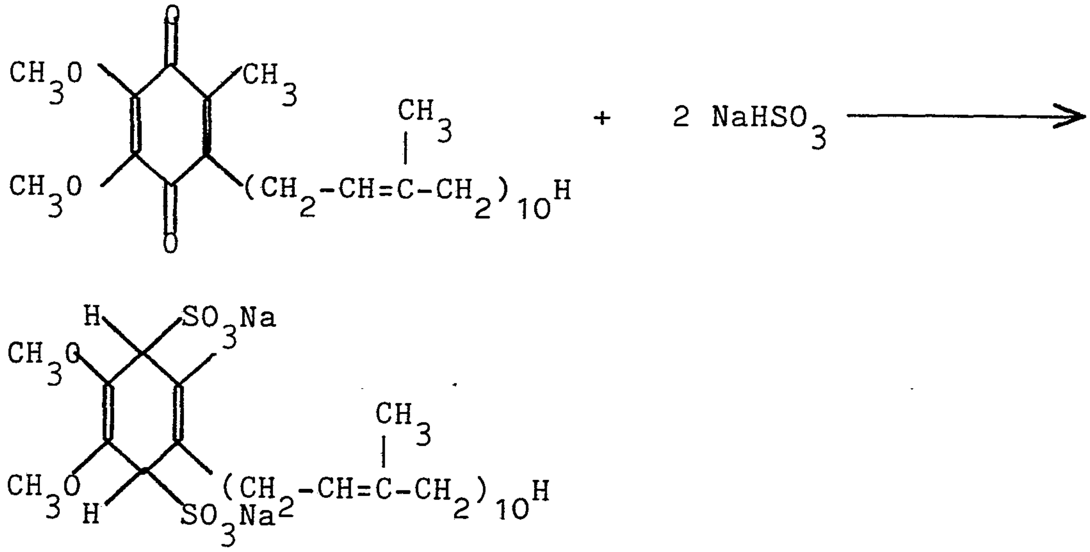


To illustrative purposes only, in the following Table the structural formulas are reported of the aminoacids within the meanings of $R_1$ and $R_2$:

TABLE

| Structural formula for $R_1$ and $R_2$ | Chemical Name |
|---|---|
| $=N-CH_2-COONa$ | (Glycine) |
| $=N-CH-COONa$ <br> $CH_2-CH(CH_3)_2$ | (L-leucine) |
| $=N-CH-COONa$ 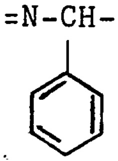 | (Phenylglycine) |
| $=N-CH-COONa$ <br> $CH_2C_6H_5$ | (Phenylalanine |

$=N-CH_2-CH_2-SO_3Na$ (Taurine)

$=N-CH(CH_2-COONa)-COONa$ (Aspartic acid)

The pharmaceutical compositions with anti-hypertensive activity which are the object of the present invention contain a therapeutically active amount of active principle dissolved in and/or admixed with a liquid or solid support normally used for these compositions.

The active principles may be administred by oral or rectal way, or they may be injected by intravenous or intramuscular way.

For the oral administering, a non-toxic pharmaceutical preparation can be formed by mixing one or more compound(s) of formula (I) with the supports normally used to that purpose, such as talc, arabic gum, sucrose, lactose, corn or wheat starch, potato flour, magnesium stearate, alginates, tragacanth gum, carrageenates, polyvinyl alcohol, polyvinylpyrrolidone, dispersing agents, emulsifiers and/or preservatives (see L.G.Goodman, A. Gilman, "The Pharmacological Basis of Therapeutics).

The end products may be used in the form of capsules, tablets, sugar-coated tablets, single-dose bags.

For the administering by the rectal way, the active principle is combined with the usual semi-synthetic glycerides, whilst in the liquid preparations there may be extemporaneous solutions or extemporaneous suspensions.

The administration of the new derivatives (1 - 10 mg/kg/day) is efficacious to the purpose of suppressing the development of the hypertension, or of reducing the advanced hypertension. These derivatives may be administered three times per day in parcelled doses, containing e.g. 5, 10, 20 mg of the active compounds in addition to

the suitable physiologically tolerable supports and excipients.

The pharmacological-biological characteristics of the compounds being the object of the present invention, i.e. their characteristics of high activity and practical absence of secondary effects have been evaluated with particular reference to the chronic toxicity, to the effects of increase of arterial pressure induced by the carotid stoppage in anaesthetized dog, to the action on the arterial pressure, always in anaesthetized dog, and to the pharmacokinetics.

Chronic toxicity

The chronic toxicity of the new derivatives has been studied by oral and intravenous way in the rats of Sprague-Dawley stock of both sexes, of body weight of about 210 g. The new derivatives, used as drops or in aqueous solution, have been administred to groups of 10 animals (5 males and 5 females). The tests have been carried out in parallel with equivalent amounts of ubiquinone (hydroalcoholic solution). The doses have been of 50 mg/kg per day for three months.

No toxic effects have been had in the case of the new derivatives, likewise to the comparison drug.

Effects of the new derivatives on the increase of the arterial pressure induced by carotid stoppage and administration of noradrenaline in anaesthetized dog.

Mongrel dogs of both sexes, having an average body weight of 13,0 - 16,0 kg have been anaesthetized by intravenous administration of Nembutal, at the dosis of 30 mg/kg, and submitted to bilateral vagotomy.

The arterial pressure is measured from the femoral arteria by means of a pressure transducer of Statham type,

connected to a pre-amplifier inserted on a Hellige polygraph.

After the basal measurements, the effect on the blood pressure induced by the stoppage of common carotid arteries for 1 minute and by the administration of noradrenaline at the dose of 1 μg/kg by intravenous way is evaluated.

The animals are subdivided into two groups of three animals each, and receive respectively the doses of 2 and 4.0 mg/kg of product by intravenous way.

The carotid stoppage and the administering of noradrenaline were repeated 5 hours after the treatment, to the purpose of evaluating the effect thereof on the increase of the arterial pressure induced in the previously described ways.

The results showed the antihypertensive effect, induced as hereinabove described, at both tested doses, even if such an effect is more evident at the highest doses.

The results obtained with the new derivatives are equivalent to those of equivalent ubiquinone doses.

Action of the new derivatives on the arterial pressure in anaesthetized dog.

Mongrel dogs of both sexes having a body weight comprised within the range of from 12.0 to 15.0 kg have been submitted to bilateral vagotomy, after having been anaesthetized with Nembutal (30 mg/kg by intravenous way).

The systemic pressure has been measured from the femoral artery by means of a Statham-type transducer, connected to a pre-amplifier in its turn inserted on a Hellige polygraph.

After having carried out the basal measurements, the treatment is carried out of the animals, which are subdivided into two groups of three animals each, and which receive

the doses of 2.0 and 4.0 mg/kg by intravenous way, respectively.
The results show that, immediately after the treatment, a small and transient increase occurs of the arterial pressure, which lasts about 3o minutes. The arterial pressure returns subsequently to normal values, and then under them, remaining at such low values for periods of from 4 to 6 hours. The results obtained with the new derivatives are equivalent to those of equivalent doses of ubiquinone.

Pharmacokinetics.

When administered at dose levels equivalent to those of ubiquinone, the new derivatives show a metabolism similar to that of ubiquinone. At time zero, inside the plasma, the new derivatives are completely transformed into ubiquinone. On oral administration, a concentration about 35% higher than that of ubiquinone administered at equivalent dose levels is obtained.
The following Examples serve to illustrate the invention without however limiting it.

Example N°1 - Ubiquinone bisulphite.

A solution of 325 g (3.12 mol) of sodium bisulphite in 565 ml of water and 175 ml of ethyl alcohol at 95% concentration is prepared. The mixture is left to rest overnight, then the formed precipitate is filtered off. To the filtrate solution 250 g of ubiquinone are added under strong stirring. After 30 minutes of stirring, a bulky precipitate is obtained.
The mixture is kept under low temperature conditions over 12 hours and is then filtered. The precipitate is washed initially with 125 ml of ethyl alcohol at 95% concentration and then for 4 times, with portions of 125

ml of diethyl ether. The product obtained is vacuum dried. An amount of about 290 g of the desired product is thus obtained.

The spectroscopic analyses confirm the structure thereof.

| Elemental analysis | C | H | S | Na |
|---|---|---|---|---|
| - Calculated | 61.16% | 8.65% | 5.98% | 4.29% |
| - Found | 61.2% | 8.68% | 5.9% | 4.23% |

Molecular weight 1071.14

Example N° 2 - Ubiquinone-bis-glycine, disodium salt.

0.010 Moles of ubiquinone and 0.022 moles of glycine sodium salt are refluxed in benzene; the water which is formed is azeotropically removed. After having allowed the reaction to proceed for 3 hours, a slightly pale solution is obtained. The mixture is filtered and the solvent is evaporated under low pressure. The residue is dissolved in methanol, and the mixture is filtered to remove the insoluble matter. Methane is evaporated and the residue is washed with petroleum ether.

The desired product is obtained with a yield of about 70%. The spectroscopic analyses confirm the structure thereof.

| Elemental analysis | C | H | N | Na |
|---|---|---|---|---|
| - Calculated | 69.40% | 9.28% | 2.74% | 4.50% |
| - Found | 69.6% | 9.31% | 2.72% | 4.45% |

Molecular weight 1021.06

Example N° 3 - Ubiquinone-bis-L-leucine, disodium salt

The process is carried out as in Example N° 2, but using 0.01 moles of ubiquinone and 0.0022 moles of the sodium salt of L-leucine. The desired product is obtained with a yield of about 70%. The spectroscopic analyses confirm the structure thereof.

| Elemental analysis | C | H | N | Na |
|---|---|---|---|---|

| | | | | |
|---|---|---|---|---|
| - Calculated | 79.90% | 9.83% | 2.396% | 3.933% |
| - Found | 80% | 9.85% | 2.35% | 3.9% |

Molecular weight 1169.

Example N° 4 - Ubiquinone-bis-phenyl-glycine, disodium salt.

The process is carried out as in Example N° 2, using 0.01 moles of ubiquinone and 0.0022 moles of phenylglycine sodium salt. The desired product is obtained with a yield of about 72%.

The spectroscopic analyses confirm the structure thereof.

| Elemental analysis | C | H | N | Na |
|---|---|---|---|---|
| - Calculated | 70.69% | 8.52% | 2.32% | 3.81% |
| - Found | 70.7% | 8.6% | 2.3% | 3.8% |

Molecular weight 1206.3

Example N° 5 - Ubiquinone-bis-phenylalanine, disodium salt.

The process is carried out as in Example N° 2, using 0.01 moles of ubiquinone and 0.022 moles of disodium salt of phenylalanine. The desired product is obtained with a yield of about 75%.

The spectroscopic analyses confirm the structure thereof.

| Elemental analyses | C | H | N | Na |
|---|---|---|---|---|
| - Calculated | 71.11% | 8.66% | 2.27% | 3.728% |
| - Found | 71.3% | 8.7% | 2.3% | 3.7% |

Molecular weight 1233.18

Example N° 6 - Ubiquinone-bis-taurine, disodium salt.

The process is carried out as in Example N° 2, using 0.01 moles of ubiquinone and 0.022 moles of sodium taurinate. The desired product is obtained with a yield of 75%.

The spectroscopic analyses confirm the structure thereof.

| Elemental analyses | C | H | N | S | Na |
|---|---|---|---|---|---|
| - Calculated | 61.45% | 8.565% | 2.428% | 5.56% | 3.987% |
| - Found | 61.5% | 8.6% | 2.4% | 5.5% | 3.95% |

Molecular weight 1153.18

Example N° 7 - Ubiquinone-bis-aspartate, tetrasodium salt.

The process is carried out as in Example N° 2, using 0.01 moles of ubiquinone and 0.022 moles of tetrasodium salt of aspartic acid. The desired product is obtained with a yield of about 80%.

The spectroscopic analyses confirm the structure thereof.

| Elemental analyses | C | H | N | Na |
|---|---|---|---|---|
| - Calculated | 62.38% | 7.17% | 2.309% | 7.52% |
| - Found | 62.3% | 7.98% | 2.28% | 7.5% |

Molecular weight 1212.98.

C l a i m s

1. A compound of general formula (I)

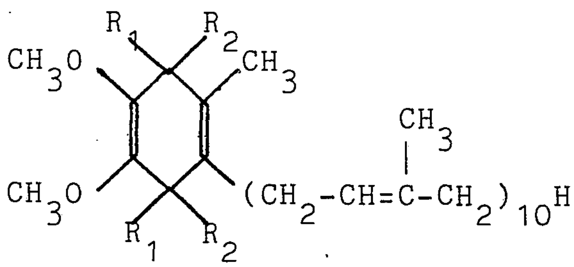

(I)

wherein $R_1$ is hydrogen and $R_2$ means $SO_3Na$, or $R_1$ and $R_2$ together mean the residue of an aminoacid selected from the group constituted by glycine, L-leucine, phenyl-glycine, phenylalanine, aspartic acid and taurine, generally in the form of an alkaline salt thereof, preferably of their sodium salt.

2. Compound according to claim 1, characterized in that it is ubiquinone bisulphite.

3. Compound according to claim 1, characterized in that it is ubiquinone-bis-glycine, disodium salt.

4. Compound according to claim 1, characterized in that it is ubiquinone-bis-L-leucine, disodium salt.

5. Compound according to claim 1, characterized in that it is ubiquinone-bis-phenylglycine, disodium salt.

6. Compound according to claim 1, characterized in that it is ubiquinone-bis-phenylalanine, disodium salt.

7. Compound according to claim 1, characterized in that it is ubiquinone-bis-taurine, disodium salt.

8. Compound according to claim 1,characterized in that it is ubiquinone-bis-aspartate, tetrasodium salt.

9. Process for the preparation of a compound of formula (I), characterized in that ubiquinone(10) is reacted with the sodium salt of an aminoacid in benzene and under refluxing conditions, in the ratio 1 : 2.2, eliminating water as an azeotrope.

10. Process for the preparation thereof, characterized in that ubiquinone(10) is reacted with an excess of sodium bisulphite in hydroalcoholic solution.

11. Pharmaceutical compositions with hypotensive action, characterized in that, as the active principle, they contain a compound according to claims from 1 to 8, together with a suitable liquid or solid support.

12. Pharmaceutical compositions having the form of tablets, sugar-coated tablets, single-dose bags, syrups and extemporaneous emulsions, suppositories, extemporaneous vials for intravenous and e.v. administering containing, as the active principle, one of the compounds according to claims from 1 to 8, singly or in combination with each other.

0200794

RACCOMANDATA

ESPRESSO

14

NOTARBARTOLO & GERVASI

STUDIO BREVETTI E MARCHI s.r.l.

Viale Bianca Maria, 33
20122 Milano (Italy)
Telephones: (02) 791969 - 799530 - 795274
Cable address: NOTAGE Milano
Telex: 330519 NOTAGE I
Telecopier: 0392 - 799530

DR. ING. M. NOTARBARTOLO
DR. G. GERVASI
PATENT AGENTS
EUROPEAN PATENT ATTORNEYS

DR. E. CASAGRANDE
DR. A. PASSINI
DR. ING. F. BARBARINO
DR. L. PICCOLO
DR. U. PALLINI
DR. G. MORETTI

EUROPEAN PATENT OFFICE
Directorate General 2
Erhardtstrasse 27
D-8000 MUENCHEN 2
West Germany

Milano July 31, 1985 Ns. Rif. CA/mb

EPA - EPO - OEB
DG 1
Reçu le
14 AUG. 1985

Dear Sirs,

RE: European Patent Application
no.85104273.9
MAGIS FARMACEUTICI
our case GIS.~~50~~ 11

REQUEST FOR CORRECTION R. 88

Please provide for the following amendments of the text and claims of the application in re.

- Page 1, line 21:
  change "hydrogen" into OH
- Page 2, line 10:
  change "H" into OH
- Page 3, lines 14-18
  change the formula

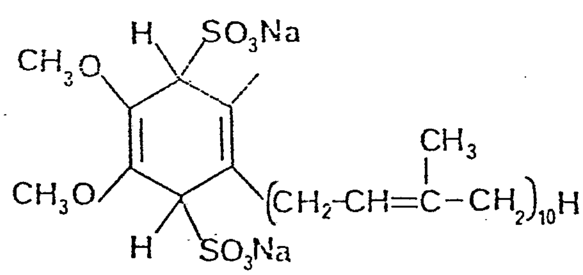

into

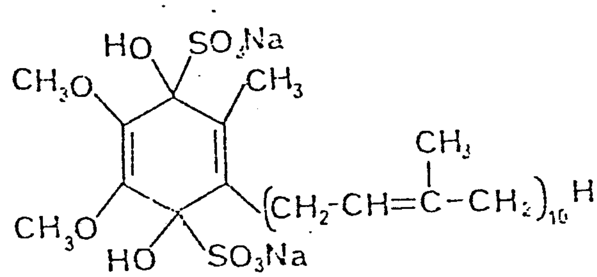

- Claim 1, line 7:
  change "hydrogen" into OH

We also inform you to note that we have changed our reference GIS.11 into GIS.30.

You will find hereto enclosed the 3 copies of the correct pages.

We look forward to hearing from you in the matter.

Yours Faithfully,

Dr.GEMMA GERVASI

Encl.

European Patent Office

# EUROPEAN SEARCH REPORT

0200794

Application number

EP 85 10 4273

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | No relevant documents have been disclosed<br>----- | | C 07 C 175/00 |
| | | | TECHNICAL FIELDS SEARCHED (Int. Cl.4) |
| | | | C 07 C 175/00 |

The present search report has been drawn up for all claims

| Place of search | Date of completion of the search | Examiner |
|---|---|---|
| THE HAGUE | 06-12-1985 | BONNEVALLE E.I.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82